# EUROPÄISCHE PATENTSCHRIFT

(11) **EP 2 111 850 B1**
(45) Veröffentlichungstag und Bekanntmachung des Hinweises auf die Patenterteilung: **19.08.2015**
(21) Anmeldenummer: 08154948.7
(22) Anmeldetag: 22.04.2008
(51) Int. Cl.: A61K 8/06, A61Q 19/00, A61K 8/37

(54) **Verwendung von Citronensäureestern als W/O-Emulgatoren**
Use of citric acid esters as W/O emulgators
Utilisation d'ésters d'acide citrique en tant qu'émulsifiants eau/huile

(43) Veröffentlichungstag der Anmeldung: 28.10.2009
(73) Patentinhaber: Azelis Deutschland Kosmetik GmbH, 47445 Moers (DE)
(72) Erfinder: Seidel, Holger, 47055, Duisburg (DE)
(74) Vertreter: Féaux de Lacroix, Stefan

(56) Entgegenhaltungen:
- GB-A- 2 361 927
- US-A1- 2002 102 342
- US-A1- 2002 146 375
- US-A1- 2007 092 478
- US-A1- 2007 207 133

## Beschreibung

Die Erfindung betrifft eine W/O-Emulsion, enthaltend mindestens eine wässrige Phase, emulgiert in mindestens einer Ölphase, und einen oder mehrere Ester von Monoglyceriden und/oder Diglyceriden von in Sonnenblumenöl vorliegenden Fettsäuren, wobei 70 bis 97,5 Gew.-% der Fettsäuren C₁₈-Fettsäuren und 2,5 bis 20 Gew.-% der Fettsäuren C₁₆-Fettsäuren sind und maximal 10 Gew.-% der Fettsäuren von diesen verschieden sind und die C₁₈-Fettsäuren Gemische aus C18:0, C18:1, C18:2 und C18:3 sind und die C₁₆-Fettsäuren Gemische aus C16:0 und C16:1 sind, mit Citronensäure, wobei die Ester ganz oder teilweise neutralisiert sein können, als W/O-Emulgator, in einer Menge im Bereich von 1 bis 20 Gew.-%, bezogen auf die gesamte Emulsion, wobei der Volumenanteil der wässrigen Phase oder Phasen in der W/O-Emulsion 50 bis 85 % beträgt sowie eine kosmetische oder pharmazeutische Zusammensetzung, enthaltend diese W/O-Emulsion.

Der Einsatz von Estern von Monoglyceriden und/oder Diglyceriden von Fettsäuren mit Citronensäure in kosmetischen Zusammensetzungen ist an sich bekannt. Beispielsweise betrifft die DE-A-103 31 760 einen O/W-Emulgator, der 70 bis 90 Gew.-% Glyceryloleatcitrat und 10 bis 30 Gew.-% eines Viskositätsmodifizierers mit einer Viskosität im Bereich von 1 bis 10000 mPas enthält. Der Emulgator wird insbesondere in kosmetischen Zusammensetzungen zur Hautpflege oder in medizinischen dermatologischen Zusammensetzungen eingesetzt.

Die DE-A 198 02 206 betrifft Lipid-reduzierte, fließfähige Zubereitungen. Die dünnflüssigen, kosmetischen oder dermatologischen Zubereitungen enthalten einen oder mehrere partiell neutralisierte Ester von Monoglyceriden und/oder Diglyceriden gesättigter Fettsäuren mit Citronensäure neben Fettalkoholen, einer Wasserphase und einer Lipidphase.

Die EP-A-1 000 603 betrifft die Verwendung von oberflächenaktiven Citronensäureestern zur Stabilisierung von Flavonen oder ähnlichen Substanzen sowie diese enthaltende kosmetische und dermatologische Zubereitungen. Dabei werden partiell neutralisierte Ester von Monoglyceriden und/oder Diglyceriden gesättigter Fettsäuren mit Citronensäure eingesetzt.

US 2007/0207133 betrifft eine Emulgator-Zusammensetzung zur Herstellung eines Wasser-in-ÖI-Produktes. Dazu wird eine hydrophobe Komponente mit einer Wasser-in-ÖI-Emulsion vermischt, um das Produkt zu bilden. Die Zusammensetzung kann eine kosmetische oder pharmazeutische Zusammensetzung sein. Grindsted^{®}Citrem 2-in-1 wird in Erdnussbutter-Zusammensetzungen eingesetzt.

GB-A-2 361 927 betrifft eine nicht gießfähige Emulsion, insbesondere ein essbares Bratfett. Das Bratfett weist in einer wässrigen Phase und in einer Fettphase jeweils Emulgatoren auf, in der wässrigen Phase Zitronensäureester von Mono- und Diglyceriden von Fettsäuren und in der Fettphase Mono- und Diglyceride von Fettsäuren. Der Wasseranteil der beispielhaften Zusammensetzungen liegt unter 30 Gew.-%.

US 2007/092478 A1 betrifft Emulsionen als kosmetische Zubereitungen mit einem Anteil an 1,2-Alkandiolen und polaren Ölkomponenten. Die Formulierungen weisen gute sensorische Eigenschaften auf, die u. a. in verbesserten in-vivo Wirksamkeiten resultieren.

US 2002/0146375 betrifft kosmetische oder pharmazeutische lecithinhaltige Gele oder O/W-Mikroemulsionen. Unter geeigneten Emulgatoren wird auch Glyceryllinoleatcitrat aufgeführt.

Aufgabe der vorliegenden Erfindung ist die Bereitstellung eines W/O-Emulgators, der in einer Ölphase löslich ist und Wassertröpfchen stabilisiert, eine niedrige Grenzflächenspannung generiert und eine mechanisch stabile Grenzfläche ausbildet. Der Emulgator soll in einem weiten Phasen-Volumen-Verhältnis von Ölphase zu wässriger Phase wirksam sein. Der Emulgator soll mit einer Vielzahl von W/O-Emulsionen verträglich sein und ein vorteilhaftes Eigenschaftsspektrum beim Einsatz in kosmetischen oder pharmazeutischen Zusammensetzungen, insbesondere kosmetischen Zusammensetzungen, zeigen. Er soll insbesondere vorteilhaft sein in kosmetischen Zusammensetzungen, die zum Auftragen auf die Haut vorgesehen sind. Eine weitere Aufgabe ist die Bereitstellung eines W/O-Emulgators, der mit einer Vielzahl von Ölen unterschiedlicher Polarität kompatibel ist. Er soll in unpolaren, polaren und gemischt-polaren Ölen einsetzbar sein.

Die Aufgaben werden erfindungsgemäß gelöst durch eine W/O-Emulsion, enthaltend mindestens eine wässrige Phase, emulgiert in mindestens einer Ölphase, und einen oder mehrere Ester von Monoglyceriden und/oder Diglyceriden von in Sonnenblumenöl vorliegenden Fettsäuren, wobei 70 bis 97,5 Gew.-% der Fettsäuren C₁₈-Fettsäuren und 2,5 bis 20 Gew.-% der Fettsäuren C₁₆-Fettsäuren sind und maximal 10 Gew.-% der Fettsäuren von diesen verschieden sind und die C₁₈-Fettsäuren Gemische aus C18:0, C18:1, C18:2 und C18:3 sind und die C₁₆-Fettsäuren Gemische aus C16:0 und C16:1 sind, mit Citronensäure, wobei die Ester ganz oder teilweise neutralisiert sein können, als W/O-Emulgator, in einer Menge im Bereich von 1 bis 20 Gew.-%, bezogen auf die gesamte Emulsion, wobei der Volumenanteil der wässrigen Phase oder Phasen in der W/O-Emulsion 50 bis 85 % beträgt.

Die Aufgaben werden erfindungsgemäß weiterhin gelöst durch eine kosmetische oder pharmazeutische Zusammensetzung, enthaltend eine W/O-Emulsion, enthaltend mindestens eine wässrige Phase, emulgiert in mindestens einer Ölphase, und einen oder mehrere Ester von Monoglyceriden und/oder Diglyceriden von in Sonnenblumenöl vorliegenden Fettsäuren, wobei 70 bis 97,5 Gew.-% der Fettsäuren C₁₈-Fettsäuren und 2,5 bis 20 Gew.-% der Fettsäuren C₁₆-Fettsäuren sind und maximal 10 Gew.-% der Fettsäuren von diesen verschieden sind und die C₁₈-Fettsäuren Gemische aus C18:0, C18:1, C18:2 und C18:3 sind und die C₁₆-Fettsäuren Gemische aus C16:0 und C16:1 sind, mit Citronensäure, wobei die Ester ganz oder teilweise neutralisiert sein können, als W/O-Emulgator, in einer Menge im Bereich von 1 bis 20 Gew.-%, bezogen auf die gesamte Emulsion, wobei der Volumenanteil der wässrigen Phase oder Phasen in der W/O-Emulsion 50 bis 85 % beträgt, wobei die kosmetische oder pharmazeutische Zusammensetzung den W/O-Emulgator, wie vorstehend genannt in einer Menge im Bereich von 1 bis 20 Gew.-% enthält, bezogen auf die gesamte Emulsion.

Es wurde erfindungsgemäß gefunden, dass die vorstehenden Ester besonders vorteilhafte W/O-Emulgatoren sind und damit erlauben, eine wässrige Phase in Tröpfchenform in einer Ölphase zu emulgieren, ohne dass es zu einer nennenswerten Koaleszenz der Tröpfchen der wässrigen Phase kommt. Die erfindungsgemäß eingesetzten Emulgatoren erlauben insbesondere die Ausbildung mechanisch stabiler Grenzflächen, so dass sehr stabile W/O-Emulsionen zugänglich sind. Dabei ist der W/O-Emulgator in einer Vielzahl von Ölen erfolgreich einsetzbar, wobei die Öle unpolar, polar oder gemischt-polar sein können,

Die erfindungsgemäß eingesetzten W/O-Emulgatoren leiten sich von Monoglyceriden und/oder Diglyceriden von in Sonnenblumenöl vorliegenden Fettsäuren ab. Die Monoglyceride und/oder Diglyceride werden dabei typischerweise aus Sonnenblumenöl gewonnen, beispielsweise aus essbarem, gereinigtem oder raffiniertem Sonnenblumenöl.

Es können erfindungsgemäß Monoglyceride, Diglyceride oder Mischungen von Monoglyceriden und Diglyceriden eingesetzt werden. Bevorzugt werden Mischungen von Monoglyceriden und Diglyceriden eingesetzt, die auch als Mono-Diglycerid bezeichnet werden. Das Verhältnis von Monoglyceriden zu Diglyceriden kann frei gewählt werden. Der Anteil beträgt damit jeweils 0 bis 100 Gew.-%. Sofern Monoglyceride und Diglyceride vorliegen, beträgt der Anteil jeweils 1 bis 99 Gew.-%, wobei sich die Gesamtmenge jeweils auf 100 Gew.-% ergänzt.

Als Fettsäurereste kommen solche Fettsäurereste in Betracht, wie sie in Sonnenblumenöl vorliegen. Im Sonnenblumenöl liegen die entsprechenden Fettsäuren in Form von Glycerinestern vor. Erfindungsgemäß können Fettsäuren beliebiger Zusammensetzung eingesetzt werden, sofern sie in dieser Form auch in Sonnenblumenöl vorliegen. Dabei sind 70 bis 97,5 Gew.-% der Fettsäuren C₁₉-Fettsäuren und 2,5 bis 15 Gew.-% der Fettsäuren C₁₆-Fettsäuren. Dabei sind maximal 10 Gew.-%, insbesondere maximal 5 Gew.-% der insgesamt in den Monoglyceriden und/oder Diglyceriden vorliegenden Fettsäuren, bzw. Fettsäurereste von C₁₆-Fettsäuren und C₁₆-Fettsäuren verschieden.

Besonders bevorzugt liegen 3 bis 11 Gew.-% der Fettsäuren als C₁₆-Fettsäuren vor und 85 bis 97 Gew.-% der Fettsäuren als C₁₆-Fettsäuren vor.

Die C₁₉-Fettsäuren sind Gemische aus C18:0, C18:1, C18:2 und C18:3. Dabei bedeutet die letzte Ziffer die Anzahl der ethylenisch ungesättigten Gruppen in den Fettsäuren. Die C₁₆-Fettsäuren sind Gemische aus C16:0 und C16:1.

Ein typisches Fettsäure-Spektrum von Sonnenblumenöl ist wie folgt zusammengesetzt (dabei sind Schwankungsbreiten angegeben sowie die häufigsten Durchschnittswerte in Gew.-% der Gesamtfettsäuren):

| | |
|---|---|
| C < 14 | < 0,4 |
| C14:0 | < 0,5 |
| C16:0 | 3 bis 10 (6,5) |
| C16:1 | < 1,0 |
| C18:0 | 1 bis 10 (5) |
| C18:1 | 14 bis 65 (24) |
| C18:2 | 20 bis 75 (63) |
| C18:3 | < 0,7 |
| C20:0 | < 1,5 |
| C20:1 | < 0,5 |

Damit weist Sonnenblumenöl als Fettsäuren-Komponenten insbesondere Ölsäure (C18:1), Linolsäure (C18:2), Stearinsäure (C18:0) und Linolensäure (C18:3) auf.

Die Monoglyceride und/oder Diglyceride sind mit Citronensäure verestert, wobei in den Estern der Anteil an Citronensäure 5 bis 25 Gew.-%, vorzugsweise 10 bis 20 Gew.-% beträgt. Der Säurewert liegt vorzugsweise im Bereich von 15 bis 25, insbesondere etwa 20. Der Verseifungswert beträgt vorzugsweise 250 bis 350, insbesondere 285 bis 315. Die Iodzahl beträgt vorzugsweise 60 bis 80, besonders bevorzugt etwa 72.

Erfindungsgemäß einsetzbare Ester können aus unterschiedlichen Quellen bezogen werden. Beispielsweise kann der Ester als Grindsted® CITREM 2-in-1 Kosher von Danisco (www.danisco.com) bezogen werden.

Die Herstellung der Ester ist durch Veresterung der Monoglyceride und/oder Diglyceride der in Sonnenblumenöl vorliegenden Fettsäuren mit Citronensäure möglich. Entsprechende Verfahren sind dem Fachmann bekannt.

Erfindungsgemäß werden die Ester als W/O-Emulgatoren eingesetzt, zur Herstellung solcher W/O-Emulsionen, wie sie in kosmetischen oder pharmazeutischen Zusammensetzungen eingesetzt werden. Dabei handelt es sich bevorzugt um hautkosmetische Zusammensetzungen oder dermatologische pharmazeutische Zusammensetzungen.

Die Ölphase kann beispielsweise ein Mineralöl, Silikonöl, Pflanzenöl oder Gemisch davon enthalten. Beispiele geeigneter pharmakologisch unbedenklicher Öle sind Silikonöle und Derivate davon, die linear oder zyklisch sein können, natürliche Esteröle wie Sonnenblumenöl, Traubenkernöl oder Olivenöl, synthetische Esteröle wie Neutralöle, die linear oder verzweigt sein können, Paraffinöle und Isoparaffinöle, Esteröle wie Citrate, Lactate, Aleate, Salicylate oder Cinnamate. Ferner können Lipide und Lipidähnliche Strukturen eingesetzt werden, beispielsweise Di- und Triglyceride der gesättigten, geradkettigen Fettsäuren mit 12 bis 30 Kohlenstoffatomen, wie Laurinsäure, Myristinsäure, Palmitinsäure, Stearinsäure, Arachinsäure, Behensäure, Lignocerinsäure, Cerotinsäure, Melesinsäure sowie deren Ester mit anderen gesättigten Fettalkoholen mit 4 bis 22, vorzugsweise 12 bis 22 Kohlenstoffatomen wie Laurylalkohol, Myristylalkohol, Cetylalkohol, Stearylalkohol, Arachidylalkohol, Behenylalkohol, gesättigten Wachsalkoholen mit 24 bis 30 Kohlenstoffatomen wie Lignocerylalkohol, Cerylalkohol, Cerotylalkohol, Myricylalkohol. Bevorzugt sind Di-, Triglyceride, Fettalkohole, deren Ester oder Ether, Wachse, Silikonöle wie Polydimethylsiloxan. Geeignete Glycerintrifettsäureester sind beispielsweise Glycerintrilaurat, Glycerintrimyristat, Glycerintripalmitat, Glycerintristearat oder Glycerintribehenat. Geeignete Wachse sind beispielsweise Cetylpalmitat und Cera Alba (gebleichtes Wachs).

Weitere geeignete Öl- und Lipidphasen sind in EP-A-1 000 603 in den Absätzen [0048] bis [0094] beschrieben.

Als wässrige Phase wird in der Regel Wasser eingesetzt. Die Wasserphase kann darüber hinaus Glycerin, Polyethylenglycol, Propylenglycol, Ethylenglycol und ähnliche Verbindungen sowie Derivate davon enthalten. Es können zudem wässrige Lösungen oder Mischungen von Wasser mit wassermischbaren Flüssigkeiten wie Glycerin, Alkoholen und anderen Polyolen eingesetzt werden. Mögliche Zusatzstoffe sind beispielsweise Glucose, Fructose, Mannose, Xylose, Mannit, Sorbit und Xylit. Ferner können Elektrolyte wie Natriumchlorid zugesetzt werden.

Die Erfindung betrifft auch eine W/O-Emulsion, enthaltend mindestens eine wässrige Phase, emulgiert in mindestens einer Ölphase, und mindestens einen W/O-Emulgator, wie er vorstehend definiert ist, wobei der Volumenanteil der wässrigen Phase oder Phasen in der W/O-Emulsion 30 bis 85 % beträgt, besonders bevorzugt 50 bis 85 % beträgt, was Viskositäten von etwa 5000 bis 300000 mPas, vorzugsweise 5800 bis 255000 mPas, entspricht.

Es handelt sich bei der kosmetischen Zusammensetzung vorzugsweise um eine Hautpflegezusammensetzung und bei der pharmazeutischen Zusammensetzung um eine dermatologische Zusammensetzung. Die kosmetische oder pharmazeutische Zusammensetzung enthält den W/O-Emulgator vorzugsweise in einer Menge im Bereich von 1 bis 20 Gew.-%, insbesondere 1 bis 5 Gew.-%, bezogen auf die gesamte Emulsion.

Die Menge kann dabei nach den praktischen Erfordernissen, u. a. auch an die Lagerstabilität der Zusammensetzungen, gewählt werden.

Die notwendige Emulgatormenge hängt auch vom Phasen-Volumen-Verhältnis und den Tröpfchengrößen zwischen Ölphase und wässriger Phase ab. Der W/O-Emulgator kann dabei in beliebigen geeigneten kosmetischen und pharmazeutischen Zusammensetzungen in Kombination mit weiteren Emulgatoren und in Kombination mit Wirkstoffen eingesetzt werden.

Zusätzlich zum W/O-Emulgator können auch weitere geeignete W/O-Emulgatoren wie Silikoncopolyole, Polyglycerylester oder Sorbitanmonolaurat eingesetzt werden. Die erfindungsgemäßen Ester machen dabei vorzugsweise 25 bis 100 Gew.-%, besonders bevorzugt 50 bis 100 Gew.-%, insbesondere 70 bis 100 Gew.-% der insgesamt eingesetzten W/O-Emulgatoren aus. Zusätzliche W/O-Emulgatoren sind dem Fachmann bekannt.

Die Zusammensetzungen können viskositätserhöhende und/oder die Rheologie modifizierende Stoffe wie Wachse, Metallseifen, Polyalkylvinylpyrrolidone, Siliconcrosspolymere, Dextrin-Derivate oder Sucroseester enthalten.

Ferner können gegebenenfalls Polyole mitverwendet werden. Geeignete Polyole sind Propylenglycol, Butylenglycol, Ethylenglycol, Polyalkylenglycol, Glycerin, Polyglycerin, Glycoside, Sorbit, Mannit, Pentaeritrit, Dimethylolpropan oder Mischungen davon. Als Polyalkylenglycole können beispielsweise Polyethylenglycol und Polypropylenglycol eingesetzt werden. Ferner können Esterpolyole mitverwendet werden, die sich von Glycerin oder Alkylenglycolen ableiten können.

In den Zusammensetzungen kann das Spreit- und Haftvermögen durch Silikonzusatz wie Dimethicon, Cyclomethicon oder Siliconderivate erhöht werden. Hierdurch kann auch die Wasserresistenz erhöht werden. Geeignete Silikonderivate sind dabei Alkylund Arylsubstituierte Silikone, Silikon-Copolyole mit Alkylpolyglycosiden, Aminosubstituierte Silikonöle, sowie andere modifizierte Silikonöle.

Erfindungsgemäß zudem verwendbare kosmetische Hilfsstoffe sind beispielsweise Konservierungsmittel, Bakterizide, Parfume, Substanzen zum Verhindern des Schäumens, Farbstoffe, Pigmente, Verdickungsmittel, oberflächenaktive Substanzen, weich machende, anfeuchtende und/oder feucht haltende Substanzen oder Komplexbildner, die schädliche Metallionen durch Komplexbildung inaktivieren.

Geeignete Komplexbildner wie auch Antioxidantien sind in der EP-A-1 000 603 beschrieben. Für Komplexbildner kann auf die Absätze [0070] bis [0074] verwiesen werden. Für Antioxidantien kann auf die Absätze [0080] bis [0081] verwiesen werden.

Die erfindungsgemäßen Zusammensetzungen können ferner Duftstoffe und Aromen enthalten.

Unter Duftstoffen und Aromen werden erfindungsgemäß sowohl Duftöle (Flagrance) wie auch Aromastoffe (Flavour) verstanden. Es handelt sich hierbei um Riechstoffe, speziell Duftstoffe. Grundstoffe der Duftstoffe sind in der Regel etherische Öle, Blütenöle, Extrakte aus pflanzlichen und tierischen Drogen, aus Naturprodukten isolierte, chemisch veränderte (halbsynthetische) sowie rein synthetisch gewonnene Riechstoffe.

Die Duftstoffe und Aromen können dabei aus einer Vielzahl pflanzlicher Ausgangsmaterialien stammen. Beispielsweise können genannt werden: Blüten, beispielsweise von Lavendel, Rosen, Jasmin, Neroli; Stängel und Blätter, beispielsweise von Geranium, Patchouli, Petitgrain, Früchte wie Anis, Koriander, Kümmel, Wacholder; Fruchtschalen, beispielsweise von Agrumen wie Bergamotte, Zitronen, Orangen; Samen wie Macis, Angelika, Sellerie, Kardamom; Wurzeln wie Angelika, Costus, Iris, Calmus; Holz wie Sandel-, Guajak-, Zedern-, Rosenholz; Kräuter und Gräser wie Estragon, Limonengras, Salbei, Thymian; Nadeln und Zweige, beispielsweise von Fichten, Tannen, Kiefern, Latschen; Harze und Balsame, beispielsweise aus Galvanum, Elemi, Benzoe, Myrrhe, Olibanum, Opoponax.

Tierische Rohstoffe sind beispielsweise Ambra, Moschus, Zibet, Castoreum.

Beispiele halb synthetischer Riechstoffe sind Isoeugenol, Vanillin, Hydroxycitronellal, Citronellol, Geranylacetat, Jonone und Methyljonone. Die vollsynthetischen Riechstoffe oder Duftstoffe sind sehr vielfältig und orientieren sich häufig an natürlichen Stoffen. Für eine Beschreibung der Duftstoffe kann beispielsweise auf Römpp, Chemielexikon, 9. Auflage, Stichworte "Parfums", "Riechstoffe", "Duftstoffe", verwiesen werden. Weitere geeignete Duftstoffe und Aromen sind dem Fachmann bekannt.

Für den Einsatz in den erfindungsgemäßen Emulsionen geeignete kosmetische Wirkstoffe sind beispielsweise Antioxidantien.

Weitere geeignete Zusatzstoffe sind Panthenol und Panthotensäure, die aus dem medizinischen Bereich zur Wundheilung sowie aus Haarbehandlungsmitteln und Futterzusätzen bekannt sind.

Weiterhin geeignet sind kosmetische Wirkstoffe, die insbesondere oxidations- oder hydrolyseempfindlich sind wie beispielsweise Polyphenole. Hier seien genannt Catechine (wie Epicatechin, Epicatechin-3-gallat, Epigallocatechin, Epigallocatechin-3-gallat), Flavonoide (wie Luteolin, Apigenin, Rutin, Quercitin, Fisetin, Kaempherol, Rhametin), Isoflavone (wie Genistein, Daidzein, Glycitein, Prunetin), Cumarine (wie Daphnetin, Umbelliferon), Emodin, Resveratrol, Oregonin.

Geeignet sind Vitamine wie Retinol, Tocopherol, Ascorbinsäure, Riboflavin, Pyridoxin.

Geeignet sind ferner Gesamtextrakte aus Pflanzen, die u.a. obige Moleküle oder Molekülklassen enthalten.

Bei der erfindungsgemäßen Zusammensetzung kann es sich um ein Lichtschutzmittel handeln. Lichtschutzmittel sind dabei beispielsweise Sonnenöle, Sonnenmilch, Sonnencreme, Sonnenlotion, Sonnensprayöl oder Sonnenspray-Emulsion.

Bei den Wirkstoffen handelt es sich gemäß einer Ausführungsform der Erfindung um Lichtschutzfilter. Diese können als organische Lichtschutzfilter bei Raumtemperatur (25°C) in flüssiger oder fester Form vorliegen. Geeignete Lichtschutzfilter (UV-Filter) sind beispielsweise Verbindungen auf Basis von Benzophenon, Diphenylcyanacrylat oder p-Aminobenzoesäure. Konkrete Beispiele sind (INCI- oder CTFA-Bezeichnungen) Benzophenone-3, Benzophenone-4, Benzophenone-2, Benzophenone-6, Benzophenone-9, Benzophenone-1, Benzophenone-11, Etocrylene, Octocrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxydibenzoylmethane, Ethylhexyl Salicylate, Homosalate sowie Methylene-Bis-Benzotriazolyl Tetramethylbutylphenol (2,2'-Methylen-bis-{6-(2H-benzoetriazol-2-yl)-4-(1,1,3,3-tetramethylbutyl)-phenol}, 2-Hydroxy-4-methoxybenzophenon-5-sulfonsäure und 2,4,6-Trianilino-p-(carbo-2'-ethylhexyl-1'-oxi)-1,3,5-triazin.

Weitere organische Lichtschutzfilter sind Octyltriazone, Avobenzone, Octylmethoxycinnamate, Octylsalicylate, Benzotriazole und Triazine. Für weitere UV-Absorber, insbesondere UVB-Filter und UVA-Filter kann auf EP-A-1 000 603, Absätze [0099] bis [0108] verwiesen werden.

In den Lichtschutzmitteln oder Sonnenschutzmitteln können ferner anorganische Pigmente wie Titandioxid- und Zinkoxid-Pigmente eingesetzt werden. Ferner können auch Zeroxid oder Zirkonoxid als UV-Absorber eingesetzt werden. Dabei kann es sich beispielsweise um Mikropigmente handeln. Die Mikropigmente oder Pigmente können zudem mit weiteren Zusatzstoffen oberflächlich beschichtet werden. In den Mikropigmenten beträgt die mittlere Teilchengröße vorzugsweise 5 bis 100 nm, besonders bevorzugt 10 bis 50 nm. Hydrophil gecoatete Mikropigmente können in die wässrige Phase eingebracht werden, hydrophob gecoatete Mikropigmente in die Ölphase.

In pharmazeutischen Zusammensetzungen kann eine Vielzahl geeigneter pharmakologisch aktiver Substanzen eingesetzt werden, die entweder in der Ölphase oder der wässrigen Phase löslich oder dispergierbar sind. Geeignete pharmakologische Wirkstoffe sind dem Fachmann bekannt.

Besonders bevorzugt handelt es sich bei den erfindungsgemäßen Zusammensetzungen um kosmetische Zusammensetzungen, die auf die Haut aufgetragen werden.

Die einzelnen Phasen der Emulsionen können noch übliche für die einzelnen Phasen bekannte Inhaltsstoffe aufweisen. Beispielsweise können die einzelnen Phasen weitere in diesen Phasen lösliche pharmazeutische oder kosmetische Wirkstoffe enthalten. Die wässrige Phase kann beispielsweise organische lösliche Lichtschutzfilter, hydrophil gecoatetes Mikropigment, Elektrolyte, Alkohole usw. enthalten. Einzelne oder alle der Phasen können zudem Feststoffe enthalten, die vorzugsweise ausgewählt sind aus Pigmenten oder Micropigmenten, Mikrosphären, Silikagel und ähnlichen Stoffen. Die Ölphase kann beispielsweise organisch modifizierte Tonmineralien, hydrophob gecoatete (Micro)Pigmente, organische öllösliche Lichtschutzfilter, öllösliche kosmetische Wirkstoffe, Wachse, Metallseifen wie Magnesiumstearat, Vaseline oder Gemische davon enthalten. Als (Micro)Pigmente können Titandioxid, Zinkoxid und Bariumsulfat sowie Wollastonit, Kaolin, Talk, Al₂O₃, Bismutoxidchlorid, micronisiertes Polyethylen, Glimmer, Ultramarin, Eosinfarben, Azofarbstoffe, genannt werden. Insbesondere Titandioxid oder Zinkoxid sind in der Kosmetik als Lichtschutzfilter gebräuchlich und lassen sich mittels der erfindungsgemäßen Emulsionen besonders glatt und gleichmäßig auf die Haut auftragen. Mikrosphären oder Silikagel können als Träger für Wirkstoffe eingesetzt werden.

Die Emulsionen oder Dispersionen können in Abhängigkeit von der Zusammensetzung, vom Phasenvolumenverhältnis und dem gegebenenfalls vorhandenen Feststoffanteil als feste oder fließfähige Emulsionen oder Dispersionen hergestellt werden und vorliegen. Es handelt sich dabei um sehr stabile Emulsionen oder Dispersionen, die unter normalen Handhabungsbedingungen eine hohe Langzeitstabilität aufweisen. Sie erfüllen insbesondere die üblichen Stabilitätsanforderungen im Temperaturbereich von -5 °C bis + 45 °C. Die in der Emulsion oder Dispersion vorliegenden Tröpfchen oder Partikel sind dabei sehr beständig, weshalb die Emulsionen oder Dispersionen insbesondere als Träger für viele Arten von Wirkstoffen geeignet sind.

Die mit Hilfe der genannten Emulgatoren hergestellten Emulsionen oder Dispersionen werden vorzugsweise mit einem Rotor/Stator-System hergestellt, wobei die Tröpfchengröße und damit die Stabilität der Emulsionen durch die eingetragene Rührwerksenergie und die Art des Rührwerkzeugs maßgeblich beeinflusst werden.

Die erfindungsgemäßen Zusammensetzungen können durch Eintrag entsprechender Wirkstoffe an die jeweilige Anwendung angepasst werden. Beispiele geeigneter, weiterer Zusatzstoffe sind Nährstoffe, Vitalstoffe, Mineralstoffe, Vitamine, Spurenelemente, biologisch wirksame Stoffe und therapeutisch wirksame Stoffe. Beispielsweise können Vitamine und Mineralstoffe enthalten sein.

Die Herstellung der Zusammensetzungen erfolgt nach bekannten Verfahren durch Vermischen der Inhaltsstoffe. Die Ausbildung der W/O-Emulsion wird typischerweise durch Einarbeiten der wässrigen Phase in die Ölphase unter Energieeintrag erreicht.

Die Erfindung wird durch die nachstehenden Beispiele näher erläutert.

### Beispiele

### Beispiel 1

Die nachstehenden Bestandteile der Phasen A und B wurden jeweils miteinander vermischt. Sodann wurde Phase B langsam unter Homogenisieren zu Phase A gegeben. Anschließend wurde für eine Minute homogenisiert.

Das Produkt wurde rheologisch untersucht, und ein Stabilitätstest wurde durchgeführt. Dabei wurde die Lagerstabilität bei Raumtemperatur, bei 40°C, 50°C bestimmt und es wurden Tau-Gefrier-Zyklen (TGC) durchgeführt. Dabei wurde im 24-Stunden-Rhythmus von -18°C auf +40°C erhitzt und wieder abgekühlt.

Die Zusammensetzungen und die Ergebnisse sind in den nachstehenden Tabellen 1 und 2 zusammengefasst.

**Tabelle 1**

| Ölphasenpolarität | | | polar | unpolar | gemischt polar | |
|---|---|---|---|---|---|---|
| **Rezepturen** | | | **Rez 1** | **Rez 2** | **Rez 3** | **Rez 4** |
| | **CTFA/INCI** | **Hersteller** | **Gew.-%** | **Gew.-%** | **Gew.-%** | **Gew.-%** |
| **Phase A** | | | | | | |
| Grindsted Citrem 2-in-1 Kosher | Glyceryl Linaoleate Citrate* | Danisco | 5,00 | 5,00 | 5,00 | 3,00 |
| Lexol IP P | Isopropyl palmitate | Inolex | 5,00 | 0,00 | 3,00 | 3,00 |
| Palmil O | Octyl palmitate | Undesa | 5,00 | 0,00 | 3,00 | 3,00 |
| Blanova Sweet Almond Oil | Prunus Amygdalus Dulcis (Sweet Almond) Oil | S.Black | 5,00 | 0,00 | 3,00 | 3,00 |
| Blanova Eveneing Pimrose Oil | Oenthera biennis | S.Black | 5,00 | 0,00 | 3,00 | 3,00 |
| Isohexadecane | Isohexadecane | Lanxess | 0,00 | 8,00 | 3,00 | 3,00 |
| Squalane | Squalane | Worlee | 0,00 | 6,00 | 2,00 | 2,00 |
| Merkur White Oil Pharma 40 PB | Paraffinum Liquidum | Merck | 0,00 | 6,00 | 3,00 | 3,00 |

| **Phase B** | | | | | | |
|---|---|---|---|---|---|---|
| demin.water | water | - | 63,00 | 63,00 | 63,00 | 65,00 |
| Glycerin | Glycerin | Merck | 5,00 | 5,00 | 5,00 | 5,00 |
| Ethanol | Ethanol | Merck | 6,00 | 6,00 | 6,00 | 6,00 |
| NaCl | Sodium Chloride | Merck | 1,00 | 1,00 | 1,00 | 1,00 |
| **Total** | | | **100,00** | **100,00** | **100,00** | **100,00** |

| | | | | | | |
|---|---|---|---|---|---|---|
| * noch nicht als INCI-Bezeichnung registriert | | | | | | |

| **Rheologie** | Rez 2 | Rez 3 |
|---|---|---|
| Fließpunkt [mPa] | 3061 | 3061 |
| Viskosität [mPas] bei 1/s | 40000 | 51000 |

**Tabelle 2**

| **Stabilitätstest** | | |
|---|---|---|
| **Rezeptur-Nr.** | **Rez 2** | **Rez 3** |
| **RT** | | |
| 1 Tag | 1 | 1 |
| 1 Woche | 1 | 1 |
| 2 Wochen | 1 | 1 |
| 3 Wochen | 1 | 1 |
| 4 Wochen | 1 | 1 |
| 2 Monate | | |
| 3 Monate | | |

| **40°C** | | |
|---|---|---|
| 1 Tag | 1 | 1 |
| 1 Woche | 1 | 1 |
| 2 Wochen | 1 | 1 |
| 3 Wochen | 1 | 1 |
| 4 Wochen | 1 | 3/O |
| 2 Monate | 1 | 4/O 1% |
| 3 Monate | 1 | - |

| **50°C** | | |
|---|---|---|
| 1 Tag | 1 | 1 |
| 1 Woche | 1 | 1 |
| 2 Wochen | 1 | 1 |
| 3 Wochen | 1 | 1 |
| 4 Wochen | 1 | 4/O 2% |
| 2 Monate | 4/O 1% | - |
| 3 Monate | - | - |

| **TGC** | | |
|---|---|---|
| 1 X | 1 | 1 |
| 2 X | 1 | 1 |
| 3 X | 1 | 1 |
| 4 X | 1 | 1 |
| 5 X | 1 | 1 |

| | | |
|---|---|---|
| 3/O: Ölabscheidung, nicht quantifizierbar 4/O: Ölabscheidung quantifizierbar 1: keine Instabilitäten | | |

### Beispiel 2

Es wurde eine W/O-Emulsion nach dem in Beispiel 1 beschriebenen Verfahren hergestellt. Dabei wurden unterschiedliche Phasen-Volumen-Verhältnisse bzw. interne Phasenvolumina eingestellt, indem die Mengen der Phasen A und B variiert wurden. Für alle Emulsionen wurden Fließpunkte und Viskositäten untersucht. Die Ergebnisse sind in der nachstehenden Tabelle 3 dargestellt.

**Tabelle 3**

| **Rezepturen** | | | **Rez 1** | **Rez 2** | **Rez 3** | **Rez 4** | **Rez 5** |
|---|---|---|---|---|---|---|---|
| | **CTFA/INCI** | **Hersteller** | **Gew.-%** | **Gew.-%** | **Gew.-%** | **Gew.-%** | **Gew.-%** |
| **Phase A** | | | | | | | |
| Miglyol 812 | Caprylic Capric Triglyceride | Sasol | 16,67 | 13,33 | 10,00 | 6,67 | 5,00 |
| Sympatens ES | Octyl Stearate | Kolb | 16,67 | 13,33 | 10,00 | 6,67 | 5,00 |
| Merkur White Oil Pharma 40 PB | Paraffinum Liquidum | Merkur | 16,67 | 13,33 | 10,00 | 6,67 | 5,00 |
| Grindsted Citrem 2-in-1 Kosher | Glyceryl Linoleate Citrate* | Danisco | 5,00 | 5,00 | 5,00 | 5,00 | |

| **Phase B** | | | | | | | |
|---|---|---|---|---|---|---|---|
| demin.water | water | - | 33,00 | 43,00 | 53,00 | 63,00 | 68,00 |
| Glycerin | Glycerin | Merck | 5,00 | 5,00 | 5,00 | 5,00 | 5,00 |
| Ethanol | Ethanol | Merck | 6,00 | 6,00 | 6,00 | 6,00 | 6,00 |
| NaCl | Sodium Chloride | Merck | 1,00 | 1,00 | 1,00 | 1,00 | 1,00 |
| **Total** | | | **100,01** | **99,99** | **100,00** | **100,01** | **100,00** |
| **Internes Phasenvolumen [%]** | | | **50** | **60** | **70** | **80** | **85** |
| **Rheologie** | | | | | | | |
| Fließpunkt [mPa] | | | / | / | / | 12200 | 24500 |
| Viskosität [mPas] bei 1/s | | | 5800 | 18500 | 43000 | 150000 | 255000 |

| | | | | | | | |
|---|---|---|---|---|---|---|---|
| * noch nicht als INCI-Bezeichnung registriert | | | | | | | |

### Beispiel 3

Es wurden W/O-Emulsionen mit unterschiedlichen Konzentrationen an Emulgator hergestellt, nach dem in Beispiel 1 beschriebenen Verfahren. Von den Emulsionen wurden Fließpunkte und Viskositäten bestimmt. Die Ergebnisse sind in der nachstehenden Tabelle 4 zusammengefasst. Sämtliche Emulsionen überstanden drei Tau-Gefrier-Zyklen ohne Entmischung.

**Tabelle 4**

| **Rezepturen** | | | **Rez 1** | **Rez 2** | **Rez 3** | **Rez 4** |
|---|---|---|---|---|---|---|
| | **CTFA/INCI** | **Hersteller** | **Gew.-%** | **Gew.-%** | **Gew.-%** | **Gew.-%** |
| **Phase A** | | | | | | |
| Miglyol 812 | Caprylic Capric Triglyceride | Sasol | 10,00 | 10,00 | 10,00 | 10,00 |
| Sympatens ES | Octyl Stearate | Kolb | 10,00 | 10,00 | 10,00 | 10,00 |
| Parafinum Perliquidum | Paraffinum Liquidum | Merkur | 10,00 | 10,00 | 10,00 | 10,00 |
| Grindsted Citrem 2-in-1 Kosher | Glyceryl Linoleate Citrate* | Danisco | 5,00 | 1,00 | 3,00 | 7,00 |

| **Phase B** | | | | | | |
|---|---|---|---|---|---|---|
| demin.water | water | - | 53,00 | 57,00 | 55,00 | 51,00 |
| Glycerin | Glycerin | Merck | 5,00 | 5,00 | 5,00 | 5,00 |
| Ethanol | Ethanol | Merck | 6,00 | 6,00 | 6,00 | 6,00 |
| NaCl | Sodium Chloride | Merck | 1,00 | 1,00 | 1,00 | 1,00 |
| **Total** | | | **100,00** | **100,00** | **100,00** | **100,00** |
| **Rheologie** | | | | | | |
| Fließpunkt [mPa] | | | | 1020 | 1020 | |
| Viskosität [mPas] bei 1/s | | | 43000 | 10000 | 30000 | 55000 |

| | | | | | | |
|---|---|---|---|---|---|---|
| * noch nicht als INCI-Bezeichnung registriert | | | | | | |

## Patentansprüche

1. W/O-Emulsion, enthaltend mindestens eine wässrige Phase, emulgiert in mindestens einer Ölphase, und einen oder mehrere Ester von Monoglyceriden und/oder Diglyceriden von in Sonnenblumenöl vorliegenden Fettsäuren, wobei 70 bis 97,5 Gew.-% der Fettsäuren C₁₈-Fettsäuren und 2,5 bis 20 Gew.-% der Fettsäuren C₁₆-Fettsäuren sind und maximal 10 Gew.-% der Fettsäuren von diesen verschieden sind und die C₁₈-Fettsäuren Gemische aus C18:0, C18:1, C18:2 und C18:3 sind und die C₁₆-Fettsäuren Gemische aus C16:0 und C16:1 sind, mit Citronensäure, wobei die Ester ganz oder teilweise neutralisiert sein können, als W/O-Emulgator, in einer Menge im Bereich von 1 bis 20 Gew.-%, bezogen auf die gesamte Emulsion, wobei der Volumenanteil der wässrigen Phase oder Phasen in der W/O-Emulsion 50 bis 85 % beträgt.

2. W/O-Emulsion nach Anspruch 1, **dadurch gekennzeichnet, dass** sie den W/O-Emulgator in einer Menge im Bereich von 1 bis 5 Gew.-% enthält, bezogen auf die gesamte Emulsion.

3. Kosmetische oder pharmazeutische Zusammensetzung, enthaltend eine W/O-Emulsion nach einem der Ansprüche 1 oder 2, wobei die kosmetische oder pharmazeutische Zusammensetzung den W/O-Emulgator, wie er in einem der Ansprüche 1 bis 2 definiert ist, in einer Menge im Bereich von 1 bis 20 Gew.-% enthält, bezogen auf die gesamte Emulsion.

## Claims

1. W/O emulsion comprising at least one aqueous phase emulsified in at least one oil phase, and one or more citric esters of monoglycerides and/or diglycerides of fatty acids present in sunflower oil, wherein 70 to 97.5% by weight of the fatty acids are C₁₈-fatty acids and 2.5 to 20% by weight of the fatty acids are C₁₆-fatty acids and at most 10% by weight of the fatty acids are different from these and the C₁₈-fatty acids are mixtures of C18:0, C18:1, C18:2 and C18:3 and the C₁₆-fatty acids are mixtures of C16:0 and C16:1, where the esters may be wholly or partially neutralized, as W/O emulsifier in an amount in the range of 1 to 20% by weight, based on the total emulsion, wherein the proportion by volume of the aqueous phase or phases in the W/O emulsion is 50 to 85%.

2. W/O emulsion according to Claim 1, **characterized in that** said emulsion comprises the W/O emulsifier in an amount in the range of 1 to 5% by weight, based on the total emulsion.

3. Cosmetic or pharmaceutical composition comprising a W/O emulsion according to either of Claims 1 or 2, wherein the cosmetic or pharmaceutical composition comprises the W/O emulsifier, which is defined in either of Claims 1 to 2, in an amount in the range of 1 to 20% by weight, based on the total emulsion.

## Revendications

1. Émulsion E/H, contenant au moins une phase aqueuse, émulsifiée dans au moins une phase huileuse, et un ou plusieurs esters de monoglycérides et/ou de diglycérides d'acides gras présents dans l'huile de tournesol, 70 à 97,5 % en poids des acides gras étant des acides gras en C₁₈ et 2,5 à 20 % en poids des acides gras étant des acides gras en C₁₆, et au plus 10 % en poids des acides gras étant différents de ceux-ci, et les acides gras en C₁₈ étant des mélanges de C18:0, C18:1, C18:2 et C18:3, et les acides gras en C₁₆ étant des mélanges de C16:0 et C16:1, avec de l'acide citrique, les esters pouvant être neutralisés en totalité ou en partie, en tant qu'émulsifiant E/H, en une quantité dans la plage allant de 1 à 20 % en poids, par rapport à l'ensemble de l'émulsion, la proportion volumique de la ou des phases aqueuses dans l'émulsion E/H étant de 50 à 85 %.

2. Émulsion E/H selon la revendication 1, **caractérisée en ce qu'**elle contient l'émulsifiant E/H en une quantité dans la plage allant de 1 à 5 % en poids, par rapport à l'ensemble de l'émulsion.

3. Composition cosmétique ou pharmaceutique, contenant une émulsion E/H selon l'une quelconque des revendications 1 ou 2, la composition cosmétique ou pharmaceutique contenant l'émulsifiant E/H, tel que défini dans l'une quelconque des revendications 1 à 2, en une quantité dans la plage allant de 1 à 20 % en poids, par rapport à l'ensemble de l'émulsion.
